# EUROPEAN PATENT APPLICATION

(11) **EP 4 850 716 A1**
(43) Date of publication of application: **09.12.2026**
(21) Application number: 25748716.5
(22) Date of filing: 30.01.2025
(51) Int. Cl.: A61M 1/36

(54) **BIOLOGICAL COMPONENT TREATMENT MATERIAL**

(30) Priority: 31.01.2024 JP 2024012400
(71) Applicant: Toray Industries, Inc., Tokyo 103-8666 (JP)
(72) Inventor: KANDA, Shungo, Otsu-shi, Shiga 520-8558 (JP); YAMASHITA, Kyohei, Otsu-shi, Shiga 520-8558 (JP); KOMACHI, Shunsuke, Otsu-shi, Shiga 520-8558 (JP); TAKAHASHI, Hiroshi, Otsu-shi, Shiga 520-8558 (JP)
(74) Representative: Kador & Partner Part mbB
(86) International application number: PCT/JP2025/002968
(87) International publication number: WO 2025/164713

(57) **Abstract**

An object of the present invention is to provide a biological component treatment material in which a decrease in biological component treatment capacity due to high-pressure steam sterilization is reduced. The present invention provides a biological component treatment material, including a water-insoluble material including: a first ligand having an amino group and an amide group; a second ligand having a hydrocarbon group optionally substituted with a hydroxy group, and an amide group; and a substrate.

## Description

### Technical Field

The present invention relates to a biological component treatment material.

### Background Art

In recent years, there have been developed techniques related to the treatment of liquids containing biological components, particularly, techniques related to blood purification therapies in which blood components such as cytokines and leukocytes are adsorbed and removed for the purpose of the treatment of inflammatory diseases, immunosuppression before and after transplantation, and the reduction of side effects such as fever and infection caused by blood products. Cytokines, which are biomarkers in inflammatory diseases, are preferred as adsorption targets, and techniques for adsorbing and removing interleukin 6 (hereinafter referred to as "IL-6") or interleukin 8 (hereinafter referred to as "IL-8"), each of which is a type of cytokines, have been developed.

Materials containing a ligand having an amide group and an amino group in a substrate have an excellent interaction with biological cells such as leukocytes and with cytokines, and biological component treatment materials using the same have been developed.

For example, Patent Literature 1 discloses a biological component treatment material which is a water-insoluble material to which a ligand having an amide group and an amino group is bound, and in which the blood purification performance is improved by controlling the content of amide groups and the content of amino groups within certain ranges.

Patent Literature 2 discloses a material for removing an activated leukocyte-activated platelet complex, which includes a water-insoluble carrier containing a ligand and a substrate, and in which the ligand has a hydrocarbon group and an amide group.

### Citation List

### Patent Literatures

Patent Literature 1: JP 6589993 B
Patent Literature 2: JP 7331368 B

### Summary of Invention

### Technical Problem

A column (hereinafter referred to as "biological component treatment column") including a biological component treatment material is mainly used for liquid treatment, and therefore, it is common to fill the column with a liquid (hereinafter referred to as "filling liquid") intended to prevent air entrapment during liquid circulation, in advance, to remove air. Since the occurrence of bacterial growth in the filling liquid at this time will have an adverse effect on the treatment of a biological component, the biological component treatment column is subjected to a sterilization treatment. A material containing a liquid can be subjected to a sterilization treatment, for example, by methods such as gamma-ray sterilization and high-pressure steam sterilization. However, high-pressure steam sterilization is preferred, because gamma-ray sterilization requires a dedicated facility for using radioactive substances.

However, it has been newly discovered in the present invention that there is a problem of a decrease in the capacity to treat a biological component (hereinafter, referred to as "biological component treatment capacity"), particularly, in the capacity to adsorb a blood component (hereinafter, referred to as "blood component adsorption capacity"), when a conventional biological component treatment material that contains a ligand having an amino group and an amide group is subjected to a high-pressure steam sterilization. When the performance decreases due to high-pressure steam sterilization, functions as the biological component treatment column cannot be achieved.

Accordingly, an object of the present invention is to provide a biological component treatment material in which a decrease in the biological component treatment capacity due to high-pressure steam sterilization is reduced.

### Solution to Problem

As a result of intensive studies to solve the above-mentioned problem, the present inventors have found the inventions according to the following (1) to (4).
(1) A biological component treatment material, including a water-insoluble material including:
   a first ligand having an amino group and an amide group;
   a second ligand having a hydrocarbon group optionally substituted with a hydroxy group, and an amide group; and
   a substrate;
   wherein the first ligand has a structure of the following general formula (I) and is bound to the substrate as indicated by the following general formula (I), and
   wherein the second ligand has a structure of the following general formula (IIa) or (IIb) and is bound to the substrate as indicated by the following general formula (IIa) or (IIb): 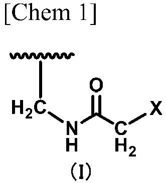 [wherein the wavy line represents a point of attachment to the substrate; and X represents a chemical structure containing an amino group as a partial structure, and optionally containing a phenyl group] 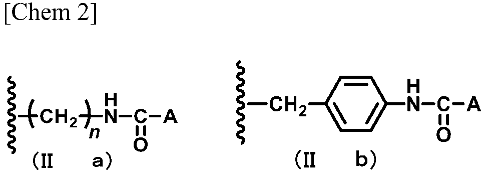 [wherein the wavy line represents a point of attachment to the substrate; A represents an alkyl group having from 1 to 6 carbon atoms or a phenyl group, optionally substituted with a hydroxy group; and n represents an integer from 1 to 6].
(2) The biological component treatment material according to (1), including the second ligand at a content of from 0.3 to 5.0 mmol per 1 g of dry weight of the water-insoluble material.
(3) The biological component treatment material according to (1) or (2), including the amino group in the first ligand at a content of from 1.0 to 4.0 mmol per 1 g of dry weight of the water-insoluble material.
(4) A biological component treatment column, including the biological component treatment material according to any one of (1) to (3).

### Advantageous Effects of Invention

The biological component treatment material according to the present invention is capable of achieving a high biological component treatment performance even when subjected to high-pressure steam sterilization.

### Description of Embodiments

The present invention will be described below in detail.

The term "substrate" refers to a material to which the first ligand and the second ligand can be immobilized by chemical modification, and which is insoluble in water after the immobilization. The substrate is, for example, a polymeric material containing in its repeating structure, a functional group having a reactivity with a carbon cation, such as an aromatic ring or a hydroxyl group. Specific examples of the substrate include synthetic polymeric materials such as: poly(aromatic vinyl compounds) (such as polystyrene and polydivinylbenzene), polyesters (such as polyethylene terephthalate and polybutylene terephthalate), polysulfone, and polyvinyl alcohol; and natural polymeric materials such as cellulose, collagen, chitin, chitosan, and dextran. The substrate may be a derivative obtained by attaching an alkyl group, a halogen group, a halogenated alkyl group, an acetal group, an ether group or the like, to any of the synthetic polymeric materials and the natural polymeric materials described above. Examples of such a derivative include polystyrene derivatives such as poly(p-chloromethylstyrene), poly(α-methylstyrene), poly(β-methylstyrene), poly(p-tert-butoxystyrene), poly(p-acetoxystyrene), and poly(p-(1-ethoxyethoxy)styrene).

The composition of the polymeric material constituting the substrate is not particularly limited. The polymeric material may be a homopolymer, or a copolymer of any of the polymeric materials described above, or alternatively, a plurality of the polymeric materials may be physically blended and used.

For use in the treatment of a blood component, in particular, the substrate is preferably a poly(aromatic vinyl compound) (such as polystyrene or polydivinylbenzene), a polyester (such as polyethylene terephthalate or polybutylene terephthalate) or a polysulfone, which is a material having no hydroxyl group, or a derivative thereof; and more preferably polystyrene, polysulfone or polydivinylbenzene, or a derivative thereof. Of these, the substrate is still more preferably polystyrene or polydivinylbenzene, or a derivative thereof, because such a polymer contains a large number of aromatic rings per unit weight, and a ligand having an amide group and an amino group can be easily introduced thereinto.

The polymeric material to be used for the substrate may include a crosslinked structure in order to exhibit water insolubility, even after the immobilization of the ligands thereto. The polymeric material including a crosslinked structure is not particularly limited. However, for example, such a polymeric material is preferably a polymeric material into which a crosslinked structure has been introduced by copolymerizing a bifunctional monomer such as divinylbenzene, or a polymeric material into which a crosslinked structure has been introduced by allowing a crosslinking agent to react with a functional group such as an aromatic ring or a hydroxyl group in the polymeric material; and more preferably a polymeric material into which a crosslinked structure has been introduced by allowing a crosslinking agent to react with a functional group such as an aromatic ring or a hydroxyl group in the polymeric material, from the viewpoint of the ease of availability. The crosslinking agent is preferably formaldehyde or a substituted benzaldehyde (such as benzaldehyde, p-diethylaminobenzaldehyde (DEAP), p-dimethylaminobenzaldehyde (DMAP) or p-isopropylbenzaldehyde (IPP)), and more preferably formaldehyde.

The term "water-insoluble material" refers to a material which is insoluble in water, and the water-insoluble material according to the present invention includes a substrate to which the first ligand and the second ligand are bound. The expression "insoluble in water" as used herein means that the change in dry weight of a material before and after placing the material in water is 1% or less. The change in dry weight as described above is the proportion of the solid component of the material, which has remained after immersing the material in 37°C-water in an amount 9 times the dry weight of the material, mixing by inversion for one hour, then withdrawing the material using forceps or the like, and vacuum drying the remaining water at 50°C or lower, with respect to the dry weight of the material before being immersed in water. If the material is not insoluble in water, there is a risk that a large amount of dissolved substances may be generated under actual use conditions, and thus is not preferred from a safety standpoint.

The term "dry weight" refers to the weight of a solid in a dry state. The expression "solid in a dry state" refers to a solid in which the amount of the liquid component contained in the solid is 1% by weight or less. When a solid is heated and dried for 24 hours at atmospheric pressure at 80°C after measuring the weight of the solid, and if the amount of decrease in weight of the remaining solid is 1% by weight or less of the weight of the solid before drying, the solid is regarded as a solid in a dry state. In the present invention, the weight refers to dry weight in all cases, unless otherwise defined.

The term "ligand" refers to a chemical structure which is contained in the water-insoluble material, and which exhibits a specific function. The water-insoluble material according to the present invention includes: a first ligand having an amino group and an amide group; and a second ligand having a hydrocarbon group optionally substituted with a hydroxy group, and an amide group; wherein the first ligand has a structure of the following general formula (I) and is bound to the substrate as indicated by the following general formula (I), and wherein the second ligand has a structure of the following general formula (IIa) or (IIb) and is bound to the substrate as indicated by the following general formula (IIa) or (IIb).

The term "first ligand" refers to a ligand which has an amino group and an amide group and which exhibits the biological component treatment capacity, and has a structure of the following general formula (I). 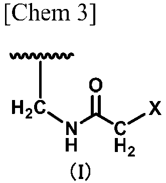 [In the general formula (I), the wavy line represents a point of attachment to the substrate; and X represents a chemical structure containing an amino group as a partial structure, and optionally containing a phenyl group].

Further, the first ligand may also have a structure of the following general formula (III), that is, a structure having an amide group, an amino group and a phenyl group. The first ligand preferably has such a structure, because the adhesion of platelets can further be inhibited. 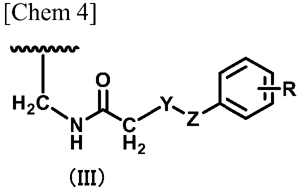 [In the general formula (III), Y represents an amino group; Z represents a linker; R represents a hydrogen atom or a halogen atom; and the wavy line represents a point of attachment to the substrate].

The amino group and the amide group are preferably covalently bound via an alkylene group, in order to control the electron density of the amino group by the amide group. The alkylene group preferably has a saturated hydrocarbon structure having 5 or less carbon atoms. The alkylene group may be for example, a pentylene group, a butylene group, a propylene group, an ethylene group or a methylene group, and more preferably a methylene group. Further, the side of the amide group, of the ligand having an amide group and an amino group described above, is bound to the substrate. The first ligand may contain a functional group other than the amide group and the amino group, and the functional group may be, for example, a phenyl group (the phenyl group may have a substituent such as a halogen atom, a halogenated alkyl group, or a linear alkyl group having from 1 to 5 carbon atoms), but not particularly limited thereto. In this case, the phenyl group is preferably bound to the amino group via a linker to be described later.

The term "amino group" refers to a chemical structure derived from an amine compound. Examples of the amino group include: amino groups derived from primary amines such as methylamine, ethylamine, propylamine, butylamine, pentylamine, hexylamine, heptylamine, octylamine and dodecylamine; amino groups derived from secondary amines such as methylhexylamine, diethylamine and dimethylamine; amino groups derived from amines having an unsaturated alkyl chain, such as allylamine; amino groups derived from tertiary amines such as trimethylamine, triethylamine, dimethylethylamine, phenyldimethylamine and dimethylhexylamine; amino groups derived from amines having an aromatic ring, such as 1-(3-aminopropyl)imidazole, pyridine-2-amine and aniline; and amino groups derived from compounds (hereinafter each referred to as "polyamine") in each of which two or more amino groups are bound via an alkyl chain, an aromatic compound, a heterocyclic compound, a homocyclic compound or the like, such as tris(2-aminoethyl)amine, ethylenediamine, diethylenetriamine, triethylenetetramine, tetraethylenepentamine, dipropylenetriamine, polyethyleneimine and N-methyl-2,2'-diaminodiethylamine. The amino group to be contained in the first ligand is preferably an amino group derived from a polyamine, more preferably an amino group derived from ethylenediamine, diethylenetriamine, triethylenetetramine, tetraethylenepentamine or polyethyleneimine, and still more preferably an amino group derived from tetraethylenepentamine or polyethyleneimine.

The number of carbon atoms per nitrogen atom in the amino group to be contained in the first ligand is preferably 18 or less, more preferably 14 or less, and still more preferably 8 or less, taking into account the nucleophilicity and the steric hindrance that affect the reaction rate. The nitrogen atom in the amino group is preferably substituted with a hydrocarbon group. The hydrocarbon group may be a linear hydrocarbon group, a branched hydrocarbon group or a cyclic hydrocarbon group, or a hydrocarbon group which is a combination of any of these. The hydrocarbon group may be, for example, an alkyl group or an alkylene group. The alkyl group is preferably a linear alkyl group such as a methyl group, an ethyl group, a propyl group, a butyl group, a pentyl group, a hexyl group, a heptyl group or an octyl group, and more preferably a methyl group, an ethyl group or a propyl group. The alkylene group is preferably a linear alkylene group such as a methylene group, an ethylene group, a propylene group, a butylene group, a hexylene group, a heptylene group or an octylene group, and more preferably a methylene group, an ethylene group or a propylene group.

The term "phenyl group" refers to a chemical structure derived from an unsubstituted or substituted benzene compound, and examples of such a compound include benzene, fluorobenzene, chlorobenzene, bromobenzene, 1,2-difluorobenzene, 1,2-dichlorobenzene, 1,2-bromobenzene, 1,3-difluorobenzene, 1,3-dichlorobenzene, 1,3-dibromobenzene, 1,4-difluorobenzene, 1,4-dichlorobenzene, and 1,4-dibromobenzene. However, the phenyl group is preferably a phenyl group derived from a halogenated benzene to which an electron-attracting group is attached, from the viewpoint of controlling the electric charge of the amino group, and in particular, the phenyl group is preferably a chlorophenyl group derived from chlorobenzene. The electron-attracting group is preferably attached to the para position, from the viewpoint of the resonance structure. Particularly in the case of a phenyl group derived from chlorobenzene, a parachlorophenyl group in which a linker and the chlorine atom are substituted at para positions to each other is preferred.

The term "halogen atom" refers to a fluorine atom, a chlorine atom, a bromine atom or an iodine atom.

The term "linker" refers to a chemical bond between the amino group and the phenyl group described above. The linker may be, for example, an electrically neutral chemical bond, such as an amide bond, a urea bond, an ether bond or an ester bond, and is preferably an amide bond or a urea bond.

The term "second ligand" refers to a ligand which has a hydrocarbon group optionally substituted with a hydroxy group, and an amide group, and which does not interfere with the biological component treatment capacity of the first ligand, and has a structure of the following general formula (IIa) or (IIb). 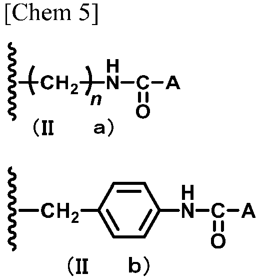 [In the general formula (IIa) or (IIb), the wavy line represents a point of attachment to the substrate; A represents an alkyl group having from 1 to 6 carbon atoms or a phenyl group, optionally substituted with a hydroxy group; and n represents an integer from 1 to 6].

When the structure of the second ligand is too long, there is a risk that the mobility may be increased, and the second ligand may interact with the first ligand, leading to a decrease in the biological component treatment capacity. Therefore, A is an alkyl group having from 1 to 6 carbon atoms or a phenyl group, optionally substituted with a hydroxy group, preferably an alkyl group having from 1 to 3 carbon atoms or a phenyl group, optionally substituted with a hydroxy group, more preferably a methyl group, a hydroxymethyl group, a phenol group or a phenyl group, and still more preferably a methyl group or a hydroxymethyl group.

The term "amide group" refers to an amide bond contained in each of the first ligand and the second ligand.

The content of amino groups in the first ligand per 1 g of dry weight of the water-insoluble material is not particularly limited. However, the content of amino groups is preferably from 1.0 to 4.0 mmol, and more preferably from 1.2 to 3.8 mmol, because too low a content results in a failure to exhibit the blood component adsorption performance, which is a type of the biological component treatment performance, and too high a content leads to a decrease in the mobility due to the density of the ligand being too high, resulting in a failure to exhibit the blood component adsorption performance.

The content of amide groups in the first ligand per 1 g of dry weight of the water-insoluble material is not particularly limited. However, the content of amide groups is preferably from 1.0 to 7.0 mmol, because too low a content results in a failure to introduce the amino group, and too high a content leads to a failure to introduce the second ligand.

The content of phenyl groups in the first ligand per 1 g of dry weight of the water-insoluble material is not particularly limited. However, the content of phenyl groups is preferably more than 0 to 7.0 mmol, because too low a content results in a failure to exhibit the effect of inhibiting platelet adhesion, and too high a content leads to a decrease in the performance to remove humoral factors from the blood.

The content of the second ligand per 1 g of dry weight of the water-insoluble material is not particularly limited. However, the content of the second ligand is preferably from 0.3 to 5.0 mmol, and more preferably from 0.4 to 3.0 mmol, because too low a content makes it more likely to cause a variation in the content of the second ligand on the surface of the water-insoluble material, and too high a content makes it more likely to cause the deformation of the material, resulting in poor handleability.

The term "biological component treatment material" refers to a material for treating a biological component, and the presence or absence of the performance for treating another substance is not particularly limited, as long as the material has the biological component treatment performance. The biological component treatment material according to the present invention is preferably a material for the adsorption and removal of a blood component. The biological component treatment material according to the present invention is required to include a water-insoluble material including the substrate, the first ligand and the second ligand, and a reinforcing material for maintaining the shape of the water-insoluble material and for allowing the material to exhibit its functions appropriately may be mixed therewith. The operation of mixing the reinforcing material may be carried out at the stage where the substrate is in a state by itself before being incorporated into the water-insoluble material, or alternatively, after binding the first ligand and the second ligand to the substrate.

The term "reinforcing material" refers to a material intentionally included in the biological component treatment material so that the shape of the water-insoluble material can be maintained and a liquid containing a biological component can be appropriately brought into contact with the water-insoluble material, and a material which does not exhibit the main biological component treatment performance on its own.

The combination of the reinforcing material and the water-insoluble material is not particularly limited. The combination may be, for example: a combination in which the island component in a sea-island composite fiber is the reinforcing material, and the sea component therein is the water-insoluble material; a combination in which one component in a polymer alloy fiber is the water-insoluble material, and another component therein is the reinforcing material; or a combination in which the nonwoven fabric in a material in the form of a nonwoven fabric is the reinforcing material, and cut fibers mixed therewith is the water-insoluble material.

The chemical structure of the reinforcing material is not particularly limited. Examples of the reinforcing material include: homopolymers each consisting of only one type of monomer selected from the group consisting of ethylene glycol, butylene glycol, terephthalic acid, aromatic vinyl compounds (such as styrene and divinylbenzene), glucose, glucose triacetate, vinylpyrrolidone, vinyl alcohol, acrylonitrile, sodium methallyl sulfonate, ethylene, propylene, ε-caprolactam and methyl methacrylate; copolymers each containing two or more types of monomers selected from the group described above; and mixtures obtained by physically blending any of the above-described homopolymers, copolymers and the like. The reinforcing material is preferably a polymer containing a compound that does not contain an aromatic ring and/or a hydroxyl group as a monomer, from the viewpoint that the adsorption by the water-insoluble material is not interfered with. Specific examples of the reinforcing material include: homopolymers each consisting of only one type of monomer selected from the group consisting of vinylpyrrolidone, acrylonitrile, sodium methallyl sulfonate, ethylene, propylene, ε-caprolactam and methyl methacrylate; copolymers each containing two or more types of monomers selected from the group described above; and mixtures obtained by physically blending any of the above-described homopolymers and copolymers. Of these, polymers (such as polyethylene and polypropylene) containing ethylene and/or polypropylene as a monomer(s) are preferred, from the viewpoint that a high chemical resistance and a high degradation resistance can be achieved. In cases where the first ligand and the second ligand are contained only in one polymer, of two or more types of polymers that are completely compatible with one another or that are contained in a block copolymer, the other polymer(s) is/are regarded as the substrate(s), and not defined as the reinforcing material(s).

The biological component treatment material may be, for example, in the form of fibers, a flat membrane, a hollow fiber membrane or particles, but is preferably in the form of fibers or particles from the viewpoint that no stagnant zone is formed during the contact with the biological component. Among those in the form of fibers described above, the biological component treatment material is preferably in the form of a yarn bundle, a yarn, a net, a knitted fabric, a woven fabric or the like, obtained by processing the fibers, and more preferably in the form of a yarn bundle, a knitted fabric or a woven fabric, in view of a large surface area and a small flow path resistance.

In cases where the biological component treatment material is in the form of particles, the particles may have an arbitrary particle size. However, the particle size is preferably from 1 µm to 1 mm, and more preferably from 20 µm to 500 µm, from the viewpoint of improving the contact area and reducing clogging.

In cases where the biological component treatment material is in the form of fibers, the single fiber diameter of the fibers may be of any size. However, the single fiber diameter is preferably from 3 to 200 µm, more preferably from 5 to 50 µm, and still more preferably from 10 to 40 µm, from the viewpoint of improving the contact area and maintaining the strength of the material.

The term "single fiber diameter" refers to the mean value of the measured values, when 10 small fiber samples are collected at random, the photograph of each sample is taken using a scanning electron microscope (SEM) or the like, and the diameter of the fiber in each photograph is measured at 10 points (a total of 100 points in all the samples).

In cases where the biological component treatment material is in the form of fibers, the fibers may have, for example, a cross-sectional structure of a single fiber composed of one type of polymer, or a core-sheath, sea-island or side-by-side composite fiber. However, a core-sheath composite fiber or a sea-island composite fiber is preferred, from the viewpoint of maintaining the strength as the material when used in the treatment of a biological component.

The biological component treatment material in the form of a knitted fabric, a felt or a net can be produced by a known method, using as a raw material, the substrate or the biological component treatment material in the form of fibers. Examples of the method of producing a felt include methods such as wet process, carding, air-laying, spun-bonding and melt blowing. Examples of the method of producing a knitted fabric or a net include methods such as plain weaving and circular knitting. In particular, a knitted fabric produced by circular knitting is preferred, from the viewpoint of having a high packing mass per unit volume, and fillability into a blood purification device.

In cases where the fibers described above have a cross-sectional structure of a sea-island solid composite fiber, the components of the fibers are not particularly limited. However, it is preferred that the sea component is the water-insoluble material and the island component is the reinforcing material, from the viewpoint of ensuring the strength while exhibiting the performance. Examples include sea-island solid composite fibers in which the sea component is composed of the water-insoluble material, and the island component is composed of a polyolefin. Specific examples include sea-island solid composite fibers in which the sea component is the water-insoluble material including polystyrene as the substrate, the first ligand and the second ligand, and the island component is composed of polypropylene.

The term "biological component" refers to a component contained in a living body, and the chemical structure or the physical structure thereof is not limited. Examples of the biological component include fat and oil components, blood components, lymphatic fluid components, viruses, and bacteria. The biological component to be treated by the biological component treatment material according to the present invention is not particularly limited. Among biological components, however, the biological component to be treated is preferably a biological component which has an electric charge that interacts with the first ligand within the structure and which is mainly present in a liquid, such as a blood component, a lymphatic fluid component, a virus or a bacterium, more preferably a blood component, and still more preferably a leukocyte component or a cytokine particularly when intended for use in the treatment of an inflammatory disease.

The term "blood component" refers to a component included in the blood, and the blood components are classified into liquid components in the blood and cells in the blood. The blood component to be treated by the biological component treatment material according to the present invention is not particularly limited, but a liquid component in the blood is preferred, among blood components.

The term "cell in the blood" refers to a cell contained in the blood, and examples thereof include: leukocyte components such as granulocytes, monocytes and lymphocytes; erythrocytes; and platelets. When intended for use in the treatment of an inflammatory disease, the blood component to be treated is preferably a leukocyte component. Among leukocyte components, preferred are monocytes and granulocytes (including activated granulocytes, activated monocytes, activated granulocyte-activated platelet complexes and activated monocyte-activated platelet complexes), which release inflammatory cytokines and the removal of which allows for controlling inflammatory diseases.

The terms "activated granulocyte" and "activated monocyte" refer to a granulocyte and a monocyte that release cytokines, active oxygen and the like in response to cytokines, Lipopolysaccharide (LPS) and the like, respectively. The degree of activation can be determined by measuring the amount of activated oxygen released by activated leukocytes, or by measuring the expression of a surface antigen by flow cytometry or the like.

The term "activated platelet" refers to a platelet that releases cytokines, active oxygen and the like in response to cytokines, LPS and the like.

The term "activated granulocyte-activated platelet complex" or "activated monocyte-activated platelet complex" refers to a complex formed by activated platelets being bound to an activated granulocyte or an activated monocyte to initiate phagocytosis of autologous tissue. Particularly, in the treatment of a patient with an inflammatory disease, it is thought that removing activated granulocyte-activated platelet complexes considered to be directly related to the pathological condition of the patient is important.

The term "liquid component in the blood" refers to an organic matter dissolved in the blood. Specific examples of the liquid component in the blood include low-molecular-weight organic compounds such as urea and amino acids; proteins such as β2-microglobulin, cytokines, IgE and IgG; and polysaccharides such as LPS. Of these, a protein such as a cytokine or a polysaccharide such as LPS is preferred as the blood component to be treated, and a cytokine is more preferred as the blood component to be treated when intended for use in the treatment of an inflammatory disease.

The term "cytokine" refers to a class of proteins that are produced by various types of cells including immunocompetent cells in response to stimuli such as infection and trauma, and released extracellularly to exert their effects. Examples of the cytokine include interferon α, interferon β, interferon γ, interleukin 1 to interleukin 15, tumor necrosis factor-α, tumor necrosis factor-β, high-mobility group box-1, erythropoietin, and monocyte chemotactic factors. Of these, interleukin 1β, IL-6, IL-8, high-mobility group box-1, tumor necrosis factor-β or the like is preferred as the blood component to be treated. In the treatment of an inflammatory disease, IL-6 or IL-8 is more preferred as the blood component to be adsorbed.

The term "treatment" refers to a process in which a specific substance is brought into contact with the water-insoluble material to undergo a change from the state before the contact. Examples of the treatment include inactivation, activation, and adsorption. From the viewpoint of treating an inflammatory disease, inactivation or adsorption is preferred, and adsorption is more preferred.

The term "inactivation" refers to a process in which a specific substance is brought into contact with the water-insoluble material to undergo a change from a state in a liquid, thereby losing its original functions. Examples of the inactivation include a process in which a protein loses its three-dimensional structure by an electrostatic interaction or a hydrophobic interaction with the surface of the water-insoluble material, thereby losing its functions to interact with other substances, or a process in which an activated leukocyte or platelet is brought into contact with the surface of the water-insoluble material to lose its activity, thereby returning to a nonactivated state.

The term "activation" refers to a process in which a specific substance is brought into contact with the water-insoluble material to undergo a change from a state in a liquid to a state where a specific function is exhibited. Examples of the activation include a process in which a denatured protein restores its three-dimensional structure by an electrostatic interaction or a hydrophobic interaction with the surface of the water-insoluble material, thereby exhibiting its functions to interact with other substances, or a process in which an inactivated leukocyte or platelet is brought into contact with the surface of the water-insoluble material, thereby turning into an activated state.

The term "adsorption" refers to a state where a specific substance is adhered to the water-insoluble material and does not come off easily. Examples of the principles of adsorption include: ionic interactions such as electrostatic interactions; adhesion by the van der Waals force, such as a hydrophobic interactions and hydrogen bonds; and biological adhesion such as adhesion of cells and phagocytosis by leukocytes.

Further, the biological component treatment material according to the present invention is preferably used as a biological component treatment column. The application of the biological component treatment column is not particularly limited, but the column is preferably used in an application for removing an unwanted biological component from a solution, and more preferably used in an application for removing a protein (such as a cytokine) from a solution. Specific examples of the application include: blood purification therapies (for example, the treatment of patients with inflammatory diseases by removing cytokines from their blood) which are applications for removing pathogenic substances from the blood; applications for removing unwanted biological components from solutions containing biological components (for example, the removal of cytokines from organ preservation solutions intended to maintain the preservation state of organs harvested for transplantation; the removal of cytokines in the production process of cell preparations such as blood products; the removal of biological components other than T-cells, during T-cell isolation in CAR-T therapies; the removal of cytokines and cell membranes from platelets derived from iPS cells; the regeneration of dialysate by treating unwanted proteins and urea in liquids such as dialysate; and the removal of unwanted proteins derived from bacteria in the production process of biopharmaceuticals). Of these, the biological component treatment column can be suitably used in a blood purification therapy in which the removal of inflammatory cytokines is effective.

In the case of using the biological component adsorption column according to the present invention as a blood purification device in a blood purification therapy, the shape of the column is not limited as long as it is a container having an inlet and an outlet through which the blood passes. Such a container may be for example, a cylindrical container, or a container in the form of a prismatic column such as a triangular prism, a quadrangular prism, a hexagonal prism or an octagonal prism. The blood drawn from the body may be directly passed through the column, or another body fluid treatment method or medical device may be used in combination. Examples of the other body fluid treatment method or medical device include plasma exchange, peritoneal dialysis, plasma separators, hemofilters, cardiopulmonary bypass, and ECMO.

The biological component treatment column according to the present invention can be suitably used in the treatment of an inflammatory disease, among blood purification therapies. When used in the treatment of an inflammatory disease, the extracorporeal circulation method is preferred in which the biological component treatment column including the biological component treatment material described above is connected to a patient through a blood circuit, a body fluid drawn from the patient is passed through the biological component treatment column, and the body fluid passed through the column is returned to the patient. A body fluid or the like is preferably treated continuously, more preferably treated for a treatment time of 4 hours or more, and still more preferably 24 hours or more, from the viewpoint of inhibiting a further induction of inflammation by blood components.

The term "inflammatory disease" refers to the entire spectrum of disease that triggers an inflammatory reaction in the body. Examples of the inflammatory disease include systemic lupus erythematosus, malignant rheumatoid arthritis, multiple sclerosis, ulcerative colitis, Crohn's disease, drug-induced hepatitis, alcoholic hepatitis, Type A hepatitis, Type B hepatitis, Type C hepatitis, Type D hepatitis, Type E hepatitis, sepsis (such as gram-negative sepsis, gram-positive sepsis, culture-negative sepsis and fungal sepsis), influenza, acute respiratory distress syndrome (ARDS), acute lung injury (ALI), pancreatitis, idiopathic pulmonary fibrosis (IPF), blood product transfusion, organ transplant, reperfusion injury after organ transplant, cholecystitis, cholangitis, and neonatal blood group incompatibility.

Among the inflammatory diseases, drug-induced hepatitis, alcoholic hepatitis, Type A hepatitis, Type B hepatitis, Type C hepatitis, Type D hepatitis, Type E hepatitis, sepsis (such as gram-negative sepsis, gram-positive sepsis, culture-negative sepsis and fungal sepsis), influenza, acute respiratory distress syndrome, acute lung injury, pancreatitis or idiopathic pulmonary fibrosis is preferred as the disease to be treated, because a causative agent is released into the blood, and a therapeutic effect by blood purification can particularly be expected. The biological component treatment column according to the present invention is preferably used in applications for the treatment of the inflammatory diseases described above. In particular, the biological component treatment column is more preferably used in applications for the treatment of sepsis (such as gram-negative sepsis, gram-positive sepsis, culture-negative sepsis and fungal sepsis), influenza, acute respiratory distress syndrome, acute lung injury and idiopathic pulmonary fibrosis, which are difficult to treat only with drugs, and thought to be diseases in which both cytokines and activated leukocyte-activated platelet complexes are involved.

The biological component treatment material according to the present invention can be produced, for example, by the following methods, but not limited thereto.

A commercially available substrate can be used as it is, as the substrate. When it is desired to mold the substrate arbitrarily in the form of fibers, a flat membrane, a hollow fiber membrane or particles, the substrate can be molded into an arbitrary form by purchasing a commercially available resin, and heat melting and extruding the resin from a die, or dissolving the resin in a good solvent and then extruding the resulting resin solution from a die into a poor solvent or into the air. As an alternative, the substrate in the form of particles can be produced by dissolving a polymeric material for forming the substrate in a good solvent, adding the resulting solution of the polymeric material dropwise into a poor solvent, and then removing the solvents to aggregate the polymer, or precipitating the polymer simultaneously with polymerization by suspension polymerization. In the case of mixing a reinforcing material into the substrate, the reinforcing material can be mixed, for example, by performing mixing and molding during the processing of the substrate, or performing physical mixing by needle punching after the processing.

The substrate containing the amide group of the first ligand as well as the second ligand can be produced by: dissolving a catalyst, a chloroacetamide having a haloalkyl group and a hydroxy group which is a compound from which the amide group of the first ligand is derived, and an acetamide having a hydrocarbon group and a hydroxy group which is a compound from which the second ligand is derived, in a non-polar solvent to prepare a solution; and adding the substrate, or the substrate and the reinforcing material, to the resulting solution to allow the reaction to proceed.

Sulfuric acid, hydrochloric acid, nitric acid, aluminum (III) chloride or iron (III) chloride is used, for example, as the catalyst, and sulfuric acid or iron (III) chloride is preferred.

N-hydroxymethyl-2-chloroacetamide (hereinafter referred to as "NMCA") can be used, for example, as the chloroacetamide having a haloalkyl group and a hydroxy group which is a compound from which the amide group of the first ligand is derived.

N-(hydroxymethyl)acetamide (hereinafter referred to as "NMA"), N-(hydroxymethyl)benzamide (hereinafter referred to as "NMBA") or N-[4-(hydroxymethyl)phenyl]acetamide (hereinafter referred to as "NMPA") can be used, for example, as the acetamide having a hydrocarbon group and a hydroxy group which is a compound from which the second ligand is derived.

As an alternative, the substrate containing the amide group of the first ligand as well as the second ligand can also be produced by: dissolving a primary amide compound having a hydrocarbon group optionally substituted with a hydroxy group, paraformaldehyde and a catalyst in a solvent, to form an acetamide having a hydrocarbon group and a hydroxy group which is a compound from which the second ligand is derived, in the system; then mixing therewith a compound from which the amide group of the first ligand is derived; and adding the substrate, or the substrate and the reinforcing material, to the resulting mixture to allow the reaction to proceed.

2-Hydroxyacetamide can be used, for example, as the primary amide compound having a hydrocarbon group optionally substituted with a hydroxy group.

Examples of the solvent include nitrobenzene, nitropropane, chlorobenzene, toluene and xylene. Of these, nitrobenzene or nitropropane is preferred.

Examples of the catalyst to be used include sulfuric acid, hydrochloric acid, nitric acid, aluminum (III) chloride and iron (III) chloride. Of these, sulfuric acid or iron (III) chloride is preferred.

In the case of introducing a crosslinking structure into the substrate, a crosslinking agent can be mixed at the same time, and paraformaldehyde (hereinafter referred to as "PFA"), acetaldehyde or benzaldehyde can be used, for example, as the crosslinking agent.

Examples of the non-polar solvent include nitrobenzene, nitropropane, chlorobenzene, toluene and xylene. Of these, nitrobenzene or nitropropane is preferred.

Commercially available products can be used as the catalyst, the chloroacetamide having a haloalkyl group and a hydroxy group which is a compound from which the amide group of the first ligand is derived, the acetamide having a hydrocarbon group and a hydroxy group which is a compound from which the second ligand is derived, the crosslinking agent and the non-polar solvent.

In the above-described reaction of the compound from which the amide group of the first ligand is derived, the compound from which the second ligand is derived and the substrate, the concentration of the catalyst in the reaction solution is preferably from 5 to 80wt%, and more preferably from 30 to 70wt%. The reaction is preferably carried out at a reaction temperature of from 0 to 90°C, and more preferably from 5 to 40°C. The reaction is preferably carried out for a reaction time of from one minute to 120 hours, and more preferably from 5 minutes to 24 hours.

The water-insoluble material including the first ligand, the second ligand and the substrate can be produced, for example, by adding the substrate containing the amide group of the first ligand as well as the second ligand to a polar solvent in which a compound (hereinafter referred to as "amine compound") having an amino group is dissolved, to allow the reaction to proceed. It is also possible to further modify the amine introduced as the first ligand, by subsequently adding the resulting material to a polar solvent in which a compound (such as chlorophenyl isocyanate) having a reactivity with an amino group is dissolved, to allow the reaction to proceed. The timing for modifying the amine compound is not particularly limited. The modification reaction may be carried out subsequently after introducing the amine compound as described above, or alternatively, the amine compound may be allowed to react with a compound having a reactivity with an amino group in advance, and then allowed to react with the substrate containing the amide group of the first ligand as well as the second ligand. In the latter case, a compound which is obtained by allowing the amine compound to react with the compound having a reactivity with an amino group and which has a modified amino group, can be introduced as the first ligand, into the substrate containing the amide group of the first ligand as well as the second ligand.

Polyethyleneimine, hexaethylenetetramine, tetraethylenepentamine, triethylenetetramine, diethylenetriamine or ethylenediamine can be used, for example, as the amine compound.

Chlorophenyl isocyanate can be used, for example, as the compound having a reactivity with an amino group.

Examples of the polar solvent include N,N-dimethylformamide, diethyl ether, dioxane, tetrahydrofuran and dimethyl sulfoxide. Of these, N,N-dimethylformamide or dimethyl sulfoxide is preferred.

In the above-described reaction of the substrate containing the amide group of the first ligand as well as the second ligand, with the compound having an amino group, the reaction temperature is preferably from 10 to 90°C, and more preferably from 30 to 60°C. The reaction time is preferably from one minute to 120 hours, and more preferably from 5 minutes to 24 hours.

The weight percentage of the water-insoluble material in the biological component treatment material can be calculated by: removing the reinforcing material from a specified amount of the biological component treatment material; measuring the weight of the water-insoluble material as the remainder; and dividing the measured weight by the weight of the biological component treatment material before the removal operation. The removal of the reinforcing material from the biological component treatment material can be achieved by physical separating the reinforcing material, when no fusion is observed in the morphological observation by visual inspection or by SEM. In the case of a biological component treatment material in which the reinforcing material is fused to a polyolefin, the remainder can be collected by immersing the biological component treatment material in xylene, and heating at the boiling point under reflux to dissolve the reinforcing material.

When a solid is heated and dried for 24 hours at atmospheric pressure at 80°C after measuring the weight of the solid, and if the amount of decrease in weight of the remaining solid is 1% by weight or less of the weight of the solid before drying, the solid can be regarded as a solid in a dry state, and the weight of the remaining solid at this time can be determined as the dry weight. In cases where the amount of decrease in weight is more than 1% by weight, it is possible to turn the solid into a dry state by heating and drying the solid for 24 hours at atmospheric pressure at 80°C, again, and repeating the process until the amount of decrease in weight is reduced below 1% by weight.

In the case of determining the content of amino groups or phenyl groups in the first ligand or the content of the second ligand, per 1 g of dry weight of the water-insoluble material, the content can be calculated by: measuring the content of amino groups or phenyl groups in the first ligand or the content of the second ligand, in the biological component treatment material after drying; and dividing the resulting value by the product (the weight of the water-insoluble material in the biological component treatment material) of the weight of the biological component treatment material and the weight percentage of the water-insoluble material in the biological component treatment material.

The content of amino groups in the first ligand per 1 g of dry weight of the water-insoluble material included in the biological component treatment material can be determined, for example, by: measuring the dry weight of the biological component treatment material after drying; ion exchanging amino groups in the biological component treatment material with hydrochloric acid; back-titrating with an aqueous solution of sodium hydroxide; and dividing the resulting value by the product of the weight of the biological component treatment material and the weight percentage of the water-insoluble material in the biological component treatment material.

The content of phenyl groups in the first ligand per 1 g of dry weight of the water-insoluble material included in the biological component treatment material can be determined, for example, by: measuring the dry weight of the biological component treatment material after drying; hydrolyzing the biological component treatment material by heating in hydrochloric acid; measuring the amount of aromatic compounds derived from phenyl groups contained in the resulting solution by ¹H-NMR; calculating the concentration of the aromatic compounds in the solution by preparing a calibration curve using an internal standard; and dividing the resulting value by the product of the weight of the biological component treatment material and the weight percentage of the water-insoluble material in the biological component treatment material.

The content of the second ligand per 1 g of dry weight of the water-insoluble material included in the biological component treatment material can be determined, for example, by: measuring the dry weight of the biological component treatment material; hydrolyzing the biological component treatment material by heating in hydrochloric acid; measuring the amount of carboxylic acids derived from the second ligand contained in the resulting solution by ¹H-NMR; calculating the concentration of the carboxylic acids in the solution by preparing a calibration curve using an internal standard; and dividing the resulting value by the product of the weight of the biological component treatment material and the weight percentage of the water-insoluble material in the biological component treatment material.

The blood component adsorption capacity of the biological component treatment material can be evaluated, for example, by a method in which: the biological component treatment material is immersed in fetal bovine serum (FBS) in which cytokines are dissolved; measuring the amount of decrease in the concentration of cytokines in FBS after immersing the treatment material therein; and calculating the adsorption rate of cytokines.

Cytokines are a class of proteins released into the blood, and it can be determined that the larger the amount of decrease in the concentration of cytokines as a result of immersing the biological component treatment material, the higher the blood component adsorption capacity of the biological component treatment material.

Since the adsorption of IL-6 or IL-8 to the biological component treatment material is considered to be an equilibrium reaction following a Langmuir model that involves a monolayer coverage on the surface, it is thought that the adsorption equilibrium is reached by performing an adsorption treatment for about 4 hours, without depending on the concentration of the adsorption target. Therefore, the IL-6 or IL-8 adsorption rate of the biological component treatment material is preferably 100% after performing an adsorption treatment for 4 hours. Since the adsorption rate is time-dependent, it can be regarded that the biological component treatment material has a sufficient IL-6 or IL-8 adsorption capacity, if the adsorption rate is 50% or more after performing an adsorption treatment for 2 hours.

The rate of decrease in the performance of the biological component treatment material after high-pressure steam sterilization can be determined, by calculating the IL-6 or IL-8 adsorption rate of the biological component treatment material before and after the high-pressure steam sterilization, and calculating as the rate of decrease in the adsorption rate before and after the high-pressure steam sterilization. A decrease in performance due to high-pressure steam sterilization is not preferred, because it leads to a failure to achieve the target performance as the biological component treatment material. In view of the variation in the adsorption of a biological component, it can be regarded that the performance is decreasing when the above-described rate of decrease in the performance before and after the high-pressure steam sterilization is 20% or more.

The pH of the filling liquid in the biological component treatment material after high-pressure steam sterilization can be determined by: performing high-pressure steam sterilization in a state where the biological component treatment material is immersed in the filling liquid; withdrawing the biological component treatment material; and measuring the pH of the filling liquid.

When the filling liquid becomes acidic or basic by subjecting the biological component treatment column to high-pressure steam sterilization, there is a risk of causing an adverse effect on the biological component to be handled, making it necessary to wash the column until the filling liquid becomes neutral. Therefore, the larger the deviation of the pH of the filling liquid after the high-pressure steam sterilization from pH 7, the longer the washing time, resulting in poor handleability. When the filling liquid becomes more acidic than pH 4 or becomes more basic than pH 11, the column is regarded to have poor handleability, because 99.9% of the filling liquid needs to be removed, or the filling liquid cannot be made neutral without neutralization.

### Examples

The biological component treatment material according to the present invention will be specifically described below with reference to Examples. However, the present invention is in no way limited to these Examples. In Examples, the symbol "wt%" represents % by weight, the unit "M" represents mol/L, and the unit "mM" represents mmol/L. The weight of the substrate, the water-insoluble material or the biological component treatment material represents dry weight, unless otherwise specified. The term "total fineness" refers to the weight (gram) of fiber per 10,000 m, and is denoted by dtex. The measurement of pH in an acid-base titration was carried out by immersing the electrode of a desktop pH meter, F-74BW (Standard ToupH Electrode 9615S-10D included) manufactured by HORIBA, Ltd. in a solution controlled to 25°C. Before the pH measurement, the pH meter was calibrated using pH Standard Solution Set [phthalate standard solution (pH: 4.01, manufactured by HORIBA, Ltd.), neutral phosphate standard solution (pH: 6.86, manufactured by HORIBA, Ltd.), and borate standard solution (pH: 9.18, manufactured by HORIBA, Ltd.)]. JNM-EZ400R (manufactured by JEOL Ltd.) was used in NMR measurements. ¹H-NMR measurements were carried out for a total of 32 scans with a relaxation time of 5 seconds, and the NMR measurements hereinafter were carried out under the same conditions, unless otherwise specified.

### < Preparation of Biological Component Treatment Materials >

### (Preparation of Substrate A)

Polystyrene as the sea component and polypropylene as the island component were separately melted and metered, allowed to flow into a spin pack incorporating a sea-island composite spinneret in which 704 distribution holes for the island component are provided per discharge hole, to form a sea-island composite flow, and melt extruded. The ratio of the island component was controlled to 50wt% to obtain a sea-island solid composite fiber having a single fiber fineness of 3.0 dtex (fiber diameter: 20 µm), and the stich length setting of a circular knitting machine (Model name: Circular Knitting Machine, MR-1, manufactured by Maruzen Sangyo Co., Ltd.) was adjusted, to obtain a circular knitted fabric (hereinafter referred to as "Substrate A") having a basis weight of 39 g/m² and a bulk density of 0.22 g/cm³. The polypropylene as the island component corresponds to the reinforcing material.

### (Preparation of Substrate B)

Fibers composed of polypropylene and having a single fiber fineness of 16 dtex were spun at a spinning speed of 1,200 m/min, and 10 of the resulting filaments were bundled to obtain a multifilament having a total fineness of 160 dtex. The resulting multifilaments were formed into a knitted fabric having a weight per unit area of 50 g/m², using the circular knitting machine in the same manner as in the preparation of the substrate 1.

Further, polysulfone pellets were dissolved in chloroform to prepare a 20% by mass polysulfone solution. The knitted fabric prepared above was immersed in the resulting solution for one hour. The substrate after the immersion was withdrawn from the solution and air dried to obtain Substrate B, which is composed of polypropylene and coated with polysulfone in an amount of 23wt% of the total weight, and which has a mass per unit area of 65 g/m². The polypropylene fibers correspond to the reinforcing material.

### (Preparation of Substrate C)

A quantity of 20 g of atactic polystyrene pellets were dissolved in 200 g of dichloromethane to prepare a polymer solution. Into a 500 mL separable flask, 300 mL of ion exchanged water was introduced, 0.5 g of polyvinyl alcohol (PVA) was added thereto, and the resulting mixture was stirred at 300 rpm using a Three-One Motor to prepare a PVA solution. Subsequently, 4 mL of the polymer solution was filled into a 5 mL syringe, the needle (inner diameter: 0.23 mm) connected to the syringe was placed in the PVA solution being stirred by the Three-One Motor, and the polymer solution was discharged over 5 minutes to reprecipitate, thereby obtaining particles. The resulting particles were stirred as-is for one hour. The resulting particles were selectively aspirated with a pipette, and collected into a centrifuge tube. In the remaining PVA solution, the operation of discharging the polymer solution and collecting the particles was repeated, until the polymer solution was depleted. The particles collected into the centrifuge tube were vacuum dried for 24 hours, to obtain 17 g of Substrate C composed of polystyrene particles. The resulting particles had a particle size of 35 µm. Substrate C contains no reinforcing material.

### (Preparation of Biological Component Treatment Material 1)

A quantity of 2.3 g of NMCA was added to a mixed solution of 31 g of nitrobenzene and 31 g of 98% by weight sulfuric acid, and the resulting mixture was stirred at 10°C until NMCA was dissolved to prepare a NMCA solution. Subsequently, 0.2 g of PFA was added to a mixed solution of 2.0 g of nitrobenzene and 2.0 g of 98% by weight sulfuric acid, and the resulting mixture was stirred at 20°C until PFA was dissolved, to prepare a PFA solution. A quantity of 4.2 g of the PFA solution cooled to 5°C was mixed with 64.3 g of the NMCA solution, the resulting mixture was stirred for 5 minutes to prepare a solution, and 1 g of Substrate A was added to the solution and immersed therein for 2 hours. Substrate A after the immersion was then immersed in nitrobenzene at 0°C to terminate the reaction, and the nitrobenzene attached to Substrate A was washed with methanol.

A quantity of 0.24 g of tetraethylenepentamine (hereinafter referred to as "TEPA") and 2.1 g of triethylamine were dissolved in 51 g of DMSO, Substrate A after washing with methanol was added to the resulting solution as it is, and immersed therein at 40°C for 3 hours. The Substrate A was filtered through a glass filter and washed with 500 mL of DMSO.

Under a nitrogen atmosphere, 0.075 g of parachlorophenyl isocyanate (hereinafter referred to as "p-Cl") was added to 47 g of DMSO that had been dehydrated and dried with activated molecular sieves 3A in advance, the mixture was heated to 30°C, and the entire amount of Substrate A after washing was immersed in the resulting mixture for one hour. Thereafter, Substrate A was filtered through a glass filter, to obtain Biological Component Treatment Material 1.

### (Preparation of Biological Component Treatment Material 2)

Biological Component Treatment Material 2 was obtained in the same manner as in the preparation of Biological Component Treatment Material 1, except that 1.725 g of NMCA and 0.575 g of NMA were used instead of 2.3 g of NMCA.

### (Preparation of Biological Component Treatment Material 3)

Biological Component Treatment Material 3 was obtained in the same manner as in the preparation of Biological Component Treatment Material 1, except that 1.725 g of NMCA and 0.575 g of NMA were used instead of 2.3 g of NMCA, TEPA was added in an amount of 0.48 g instead of 0.24 g, and p-Cl was added in an amount of 0.15 g instead of 0.075 g.

### (Preparation of Biological Component Treatment Material 4)

Biological Component Treatment Material 4 was obtained in the same manner as in the preparation of Biological Component Treatment Material 1, except that 1.725 g of NMCA and 0.575 g of NMA were used instead of 2.3 g of NMCA, TEPA was added in an amount of 0.96 g instead of 0.24 g, and p-Cl was added in an amount of 0.3 g instead of 0.075 g.

### (Preparation of Biological Component Treatment Material 5)

Biological Component Treatment Material 5 was obtained in the same manner as in the preparation of Biological Component Treatment Material 1, except that 1.15 g of NMCA and 1.15 g of NMA were used instead of 2.3 g of NMCA.

### (Preparation of Biological Component Treatment Material 6)

Biological Component Treatment Material 6 was obtained in the same manner as in the preparation of Biological Component Treatment Material 1, except that 2.3 g of NMA was used instead of 2.3 g of NMCA. In Biological Component Treatment Material 6, the first ligand is not bound to the substrate, since NMCA, which is a partial structure of the first ligand, was not added.

### (Preparation of Biological Component Treatment Material 7)

Biological Component Treatment Material 7 was obtained in the same manner as in the preparation of Biological Component Treatment Material 1, except that 0.575 g of NMCA and 1.725 g of NMA were used instead of 2.3 g of NMCA, 0.48 g of PEI was used instead of 0.24 g of TEPA, and p-Cl was added in an amount of 0.15 g instead of 0.075 g.

### (Preparation of Biological Component Treatment Material 8)

Biological Component Treatment Material 8 was obtained in the same manner as in the preparation of Biological Component Treatment Material 1, except that 2.3 g of NMCA and 0.46 g of NMBA were used instead of 2.3 g of NMCA.

### (Preparation of Biological Component Treatment Material 9)

Biological Component Treatment Material 9 was obtained in the same manner as in the preparation of Biological Component Treatment Material 1, except that 2.3 g of NMCA and 0.46 g of NMPA were used instead of 2.3 g of NMCA.

### (Preparation of Biological Component Treatment Material 10)

Biological Component Treatment Material 10 was obtained in the same manner as in the preparation of Biological Component Treatment Material 2, except that Substrate B was used instead of Substrate A.

### (Preparation of Biological Component Treatment Material 11)

Biological Component Treatment Material 11 was obtained in the same manner as in the preparation of Biological Component Treatment Material 1, except that 0.48 g of PEI was used instead of 0.24 g of TEPA, and p-Cl was added in an amount of 0.15 g instead of 0.075 g.

### (Preparation of Biological Component Treatment Material 12)

Biological Component Treatment Material 12 was obtained in the same manner as in the preparation of Biological Component Treatment Material 1, except that 2.3 g of N-(hydroxymethyl)nicotinamide and 1.15 g of NMA were used instead of 2.3 g of NMCA.

### (Preparation of Biological Component Treatment Material 13)

A quantity of 1.7 g of NMCA was added to a mixed solution of 31 g of nitrobenzene and 31 g of 98% by weight sulfuric acid, and the resulting mixture was stirred at 10°C until NMCA was dissolved to prepare a NMCA solution. Subsequently, 0.6 g of PFA was added to a mixed solution of 4.0 g of nitrobenzene and 4.0 g of 98% by weight sulfuric acid, the resulting mixture was stirred at 20°C until PFA was dissolved, and 0.6 g of 2-hydroxyacetamide was further added thereto to allow the reaction to proceed, thereby preparing a 2-hydroxy-N-(hydroxymethyl)acetamide/PFA mixed solution. A quantity of 9.2 g of the 2-hydroxy-N-(hydroxymethyl)acetamide/PFA solution cooled to 5°C was mixed with 63.7 g of the NMCA solution, the resulting mixture was stirred for 5 minutes to prepare a solution, and 1 g of Substrate A was added to the solution and immersed therein for 2 hours. Substrate A after the immersion was then immersed in 200 mL of nitrobenzene at 0°C to terminate the reaction, and the nitrobenzene attached to Substrate A was washed with methanol.

A quantity of 0.24 g of TEPA and 2.1 g of triethylamine were dissolved in 51 g of DMSO, Substrate A after washing with methanol was added to the resulting solution as it is, and immersed therein at 40°C for 3 hours. The Substrate A was filtered through a glass filter and washed with 500 mL of DMSO.

Under a nitrogen atmosphere, 0.075 g of p-Cl was added to 47 g of DMSO that had been dehydrated and dried with activated molecular sieves 3A in advance, the mixture was heated to 30°C, and the entire amount of Substrate A after washing was immersed in the resulting mixture for one hour. Thereafter, Substrate A was filtered through a glass filter, to obtain Biological Component Treatment Material 13.

### (Preparation of Biological Component Treatment Material 14)

Biological Component Treatment Material 14 was obtained in the same manner as in the preparation of Biological Component Treatment Material 13, except that N-hydroxyoctanamide was used instead of 2-hydroxyacetamide.

### (Preparation of Biological Component Treatment Material 15)

Biological Component Treatment Material 15 was obtained in the same manner as in the preparation of Biological Component Treatment Material 13, except that 2-(4-butoxyphenyl)acetohydroxamic acid was used instead of 2-hydroxyacetamide.

### (Preparation of Biological Component Treatment Material 16)

Biological Component Treatment Material 16 was obtained in the same manner as in the preparation of Biological Component Treatment Material 1, except that Substrate C was used instead of Substrate A, and 1.725 g of NMCA and 0.575 g of NMA were used instead of 2.3 g of NMCA.

### < Evaluation Methods >

The methods for evaluating each biological component treatment material will be described below.

### (Measurement of Weight Percentage of Water-Insoluble Material Included in Biological Component Treatment Material)

About 5.0 g of each biological component treatment material is cut, and the weight of the treatment material is accurately measured. The biological component treatment material and 100 mL of xylene are added to a 200 mL eggplant flask, and heated at 160°C under reflux for 24 hours to remove polypropylene added as the reinforcing material. To the resulting solution after reflux, 2 L of xylene heated to 100°C is added immediately, only insoluble components are filtered as they are through a filter paper, washed with methanol, and left to stand in a dryer at 80°C for 48 hours to obtain a water-insoluble material. The resulting water-insoluble material is dried, the weight of the water-insoluble material is measured, and the weight percentage of the water-insoluble material is determined using the following equation 1. 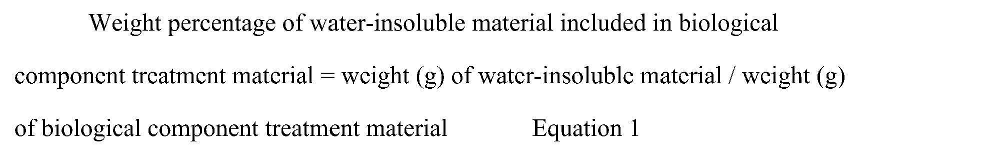 Weight percentage of water-insoluble material included in biological component treatment material = weight (g) of water-insoluble material / weight (g) of biological component treatment material Equation 1

### (Measurement of Content of Amino Groups in First Ligand in Water-Insoluble Material Included in Biological Component Treatment Material)

To a polypropylene container, 1.0 g of each biological component treatment material and 50 mL of a 6 M aqueous solution of sodium hydroxide are added, followed by stirring for 30 minutes, and the biological component treatment material is filtered using a filter paper. Subsequently, the filtered biological component treatment material is added to 50 mL of ion exchanged water, followed by stirring for 30 minutes, and the treatment material is filtered using a filter paper. The addition of the biological component treatment material to ion exchanged water and the filtration are repeated until the pH of the ion exchanged water to which the biological component treatment material has been added reaches 7, to obtain a desalted biological component treatment material. After allowing the desalted biological component treatment material to stand at 80°C under normal pressure conditions for 48 hours, 1.0 g of the biological component treatment material and 30 mL of 0.1 M hydrochloric acid are added to the polypropylene container, followed by stirring for 10 minutes. After stirring, only 5 mL of the solution is withdrawn, and transferred to another polypropylene container. Subsequently, 0.1 mL of a 0.1 M aqueous solution of sodium hydroxide is added dropwise to the resulting solution. After the dropwise addition, the resulting mixture is stirred for 10 seconds using a vortex, and the pH of the solution is measured. The stirring after the dropwise addition and the measurement of pH are repeated 50 times in the same manner. The amount of dropwise addition of the aqueous solution of sodium hydroxide when the pH of the solution exceeds 8.5 is determined as the titer. The value obtained by the following equation 2 using the titer is rounded to one decimal place, to calculate the content of amino groups in the first ligand per 1 g of dry weight of the water-insoluble material. 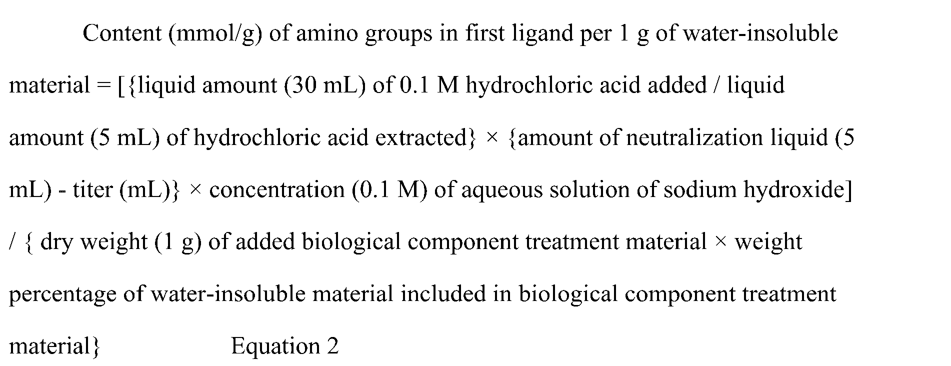 Content (mmol/g) of amino groups in first ligand per 1 g of water-insoluble material = [{liquid amount (30 mL) of 0.1 M hydrochloric acid added / liquid amount (5 mL) of hydrochloric acid extracted} × {amount of neutralization liquid (5 mL) - titer (mL)} × concentration (0.1 M) of aqueous solution of sodium hydroxide] / { dry weight (1 g) of added biological component treatment material × weight percentage of water-insoluble material included in biological component treatment material} Equation 2

### (Measurement of Content of Second Ligand in Water-Insoluble Material Included in Biological Component Treatment Material)

The content of the second ligand in the water-insoluble material included in the biological component treatment material is measured by hydrolyzing the biological component treatment material, and quantifying the amount of degradation products derived from the second ligand. The details will be described below.

In cases where the biological component treatment material is in the form of a knitted fabric (in the case of the biological component treatment material prepared using Substrate A or B), the biological component treatment material is cut into six squares each with an area of 2 cm²; and in case where the biological component treatment material is in the form of particles (in the case of the biological component treatment material prepared using Substrate C), 1 mL of the biological component treatment material is aspirated with a pipette. Each biological component treatment material that has been collected is dried, and the dry weight thereof is measured. Thereafter, the biological component treatment material after the measurement of dry weight is placed in a pressure-resistant glass bottle, and heated at 110°C for 20 hours. After heating for 20 hours, 0.99 mL of the solution in the pressure-resistant glass bottle is collected, and 0.01 mL of phenol as an internal standard is added thereto, to prepare a measurement solution. To the measurement solution, a 6 M aqueous solution of sodium hydroxide is added until the pH exceeds 7.0, in an amount of 0.05 mL at a time. A quantity of 0.1 mL of the resulting solution is withdrawn, and mixed with 10 mg of sodium trimethylsilylpropanesulfonate (hereinafter referred to as "DSS") and 0.6 mL of deuterium oxide to measure ¹H-NMR. The peak derived from DSS is set to 0 ppm, and the integral of phenol peaks (δ = 7.2 to 7.3 ppm) is normalized to 2 to calculate the integral of the peaks derived from the second ligand, and the value obtained by the following equation 3 is rounded to one decimal place, to calculate the content of the second ligand per 1 g of dry weight of the water-insoluble material. 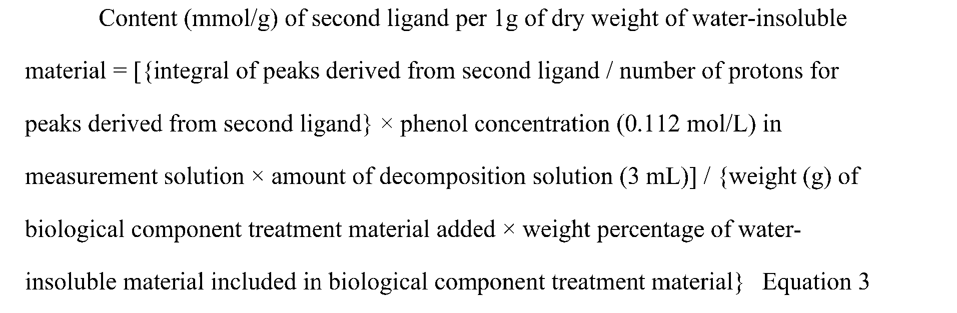 Content (mmol/g) of second ligand per 1g of dry weight of water-insoluble material = [{integral of peaks derived from second ligand / number of protons for peaks derived from second ligand} × phenol concentration (0.112 mol/L) in measurement solution × amount of decomposition solution (3 mL)] / {weight (g) of biological component treatment material added × weight percentage of water-insoluble material included in biological component treatment material} Equation 3

### (Measurement of IL-6 Adsorption Rate of Biological Component Treatment Material)

To confirm the IL-6 adsorption capacity of the biological component treatment material, the biological component treatment material is immersed in a liquid containing IL-6 for a predetermined period of time and then withdrawn therefrom, and the IL-6 adsorption rate is determined from the difference in the amount of IL-6 in the liquid before and after the immersion. The measurement method is shown below.

In cases where the biological component treatment material is in the form of a knitted fabric, the biological component treatment material is cut into four disks each with a diameter of 6 mm, the thickness of each disk is measured using a micrometer to calculate the total volume of the four disks, and all the disks are placed in a polypropylene container. In cases where the biological component treatment material is in the form of particles, 0.05 mL of the biological component treatment material is aspirated with a pipette, and the entire amount of the particles is placed in a polypropylene container. FBS prepared to an IL-6 concentration of 2,000 pg/mL is added to the container in a solid-liquid ratio of 30 mL to 1 cm³ of the total volume of the biological component treatment material. After mixing by inversion in an incubator at 37°C for 2 hours, the IL-6 concentration in FBS is measured by the ELISA method. The value obtained by the following equation 4 from the IL-6 concentrations before and after mixing by inversion is rounded to the nearest whole number, to calculate the IL-6 adsorption rate. The measurement of the absorbance is carried out using a microplate reader (Spectra Max M5; manufactured by Molecular Devices LLC), with the measurement wavelength set to 450 nm and the reference wavelength set to 595 nm, after performing a blank measurement in advance. 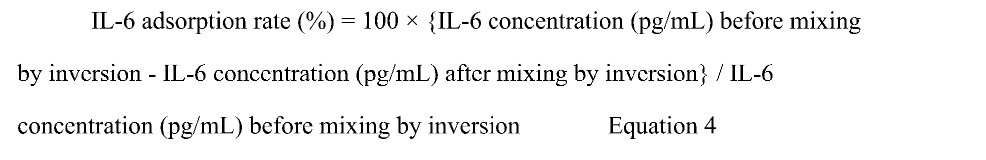 IL-6 adsorption rate (%) = 100 × {IL-6 concentration (pg/mL) before mixing by inversion - IL-6 concentration (pg/mL) after mixing by inversion} / IL-6 concentration (pg/mL) before mixing by inversion Equation 4

### (Measurement of Rate of Decrease in IL-6 Adsorption Performance of Biological Component Treatment Material before and after High-Pressure Steam Sterilization)

To a 15 mL glass vial, physiological saline is added in an amount of 10 mL with respect to 1 g of dry weight of the biological component treatment material, and the biological component treatment material is subjected to high-pressure steam sterilization using a high-pressure steam sterilizer (manufactured by TOMY SEIKO Co., Ltd.), at a sterilization temperature of 117°C for a sterilization time of 105 minutes. The IL-6 adsorption rate after the high-pressure steam sterilization is determined in the same manner as in the section of "Measurement of IL-6 Adsorption Rate of Biological Component Treatment Material" described above, except for using the biological component treatment material after the high-pressure steam sterilization thus obtained. Further, the value obtained by the following equation 5 is rounded to the nearest whole number, to calculate the rate of decrease in IL-6 adsorption performance. 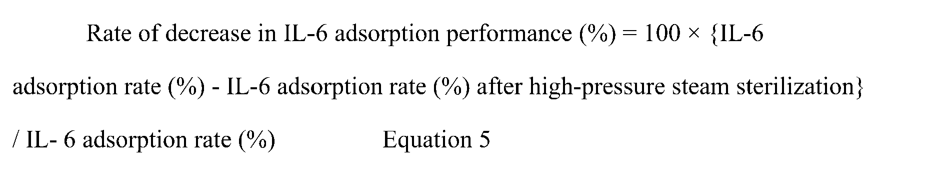 Rate of decrease in IL-6 adsorption performance (%) = 100 × {IL-6 adsorption rate (%) - IL-6 adsorption rate (%) after high-pressure steam sterilization} / IL- 6 adsorption rate (%) Equation 5

### (Measurement of IL-8 Adsorption Rate of Biological Component Treatment Material)

The IL-8 adsorption rate is determined by rounding the value obtained by the following equation 6 to the nearest whole number, in the same manner as in the section of "Measurement of IL-6 Adsorption Rate of Biological Component Treatment Material" described above, except for using IL-8 instead of IL-6. 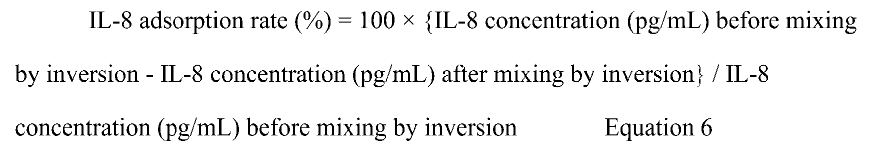 IL-8 adsorption rate (%) = 100 × {IL-8 concentration (pg/mL) before mixing by inversion - IL-8 concentration (pg/mL) after mixing by inversion} / IL-8 concentration (pg/mL) before mixing by inversion Equation 6

### (Measurement of Rate of Decrease in IL-8 Adsorption Performance of Biological Component Treatment Material before and after High-Pressure Steam Sterilization)

The IL-8 adsorption rate of the biological component treatment material after the high-pressure steam sterilization is determined in the same manner as in the measurement of the rate of decrease in IL-6 adsorption performance of the biological component treatment material before and after the high-pressure steam sterilization, except for using IL-8 instead of IL-6. Further, the value obtained by the following equation 7 is rounded to the nearest whole number, to calculate the rate of decrease in IL-8 adsorption performance. 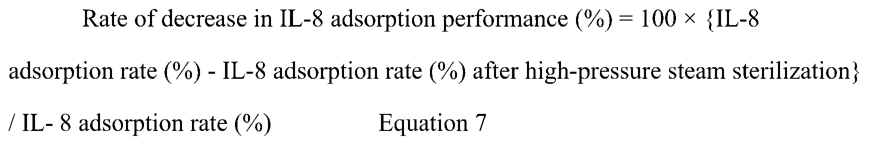 Rate of decrease in IL-8 adsorption performance (%) = 100 × {IL-8 adsorption rate (%) - IL-8 adsorption rate (%) after high-pressure steam sterilization} / IL-8 adsorption rate (%) Equation 7

### (Measurement of pH of Filling Liquid after High-Pressure Steam Sterilization of Biological Component Treatment Material)

To a 15 mL glass vial, physiological saline is added in an amount of 10 mL with respect to 1 g of dry weight of the biological component treatment material, and the biological component treatment material is subjected to high-pressure steam sterilization using a high-pressure steam sterilizer (manufactured by TOMY SEIKO Co., Ltd.), at a sterilization temperature of 117°C for a sterilization time of 105 minutes. The biological component treatment material is withdrawn from the glass vial, the pH of the remaining solution is measured, the measured value is rounded to one decimal place, and the resulting value is defined as the pH of the filling liquid after the high-pressure steam sterilization.

### < Evaluation of Biological Component Treatment Materials >

### (Measurement of Weight Percentage of Water-Insoluble Material Included in Each of Biological Component Treatment Materials 1 to 16)

The weight percentage of the water-insoluble material included in each of the biological component treatment materials 1 to 16 was determined, in accordance with operations in the section of "Measurement of Weight Percentage of Water-Insoluble Material Included in Biological Component Treatment Material" described above.

### (Measurement of Content of Amino Groups in First Ligand in Water-Insoluble Material Included in Each of Biological Component Treatment Materials 1 to 16)

The content of amino groups in the first ligand in the water-insoluble material included in each of the biological component treatment materials 1 to 16 was determined, in accordance with operations in the section of "Measurement of Content of Amino Groups in First Ligand in Water-Insoluble Material Included in Biological Component Treatment Material" described above. The measured results for the biological component treatment materials 1 to 10 are shown in Table 1, and the measured results for the biological component treatment materials 11 to 16 are shown in Table 2.

### (Measurement of Content of Second Ligand in Water-Insoluble Material Included in Each of Biological Component Treatment Materials 1 to 16)

For each of the biological component treatment materials 1 and 11, the content of the second ligand was taken as 0 mmol/g, since no compound from which the second ligand is derived was added. For each of the biological component treatment materials 2 to 7, 9, 10, 12 and 16, the content of the second ligand in the water-insoluble material included in each of the biological component treatment materials 2 to 7, 9, 10, 12 and 16 was determined in accordance with the operations in the section of "Measurement of Content of Second Ligand in Water-Insoluble Material Included in Biological Component Treatment Material" described above, assigning the peaks in the range of 1.9 to 2.1 ppm to the second ligand and setting the number of protons for the peaks derived from the second ligand to be 3. For the biological component treatment material 8, the content of the second ligand in the water-insoluble material included in the biological component treatment material 8 was determined in accordance with the operations in the section of "Measurement of Content of Second Ligand in Water-Insoluble Material Included in Biological Component Treatment Material" described above, assigning the peaks in the range of 8.0 to 8.2 ppm to the second ligand and setting the number of protons for the peaks derived from the second ligand to be 2. For the biological component treatment material 13, the content of the second ligand in the water-insoluble material included in the biological component treatment material 13 was determined in accordance with the operations in the section of "Measurement of Content of Second Ligand in Water-Insoluble Material Included in Biological Component Treatment Material" described above, assigning the peaks in the range of 4.2 to 4.3 ppm to the second ligand and setting the number of protons for the peaks derived from the second ligand to be 2. For the biological component treatment material 14, the content of the second ligand in the water-insoluble material included in the biological component treatment material 14 was determined in accordance with the operations in the section of "Measurement of Content of Second Ligand in Water-Insoluble Material Included in Biological Component Treatment Material" described above, assigning the peaks in the range of 0.7 to 0.9 ppm to the second ligand and setting the number of protons for the peaks derived from the second ligand to be 3. For the biological component treatment material 15, the content of the second ligand in the water-insoluble material included in the biological component treatment material 15 was determined in accordance with the operations in the section of "Measurement of Content of Second Ligand in Water-Insoluble Material Included in Biological Component Treatment Material" described above, assigning the peaks in the range of 7.2 to 8.1 ppm to the second ligand and setting the number of protons for the peaks derived from the second ligand to be 4. The results for the biological component treatment materials 1 to 10 are shown in Table 1, and the results for the biological component treatment materials 11 to 16 are shown in Table 2.

### (Example 1)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 2, in accordance with the operations in the sections of "Measurement of IL-6 Adsorption Rate of Biological Component Treatment Material", "Measurement of Rate of Decrease in IL-6 Adsorption Performance of Biological Component Treatment Material before and after High-Pressure Steam Sterilization", "Measurement of IL-8 Adsorption Rate of Biological Component Treatment Material" "Measurement of Rate of Decrease in IL-8 Adsorption Performance of Biological Component Treatment Material before and after High-Pressure Steam Sterilization" and "Measurement of pH of Filling Liquid after High-Pressure Steam Sterilization of Biological Component Treatment Material", respectively. The results are shown in Table 3.

### (Example 2)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 3, in the same manner as in Example 1. The results are shown in Table 3.

### (Example 3)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 4, in the same manner as in Example 1. The results are shown in Table 3.

### (Example 4)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 5, in the same manner as in Example 1. The results are shown in Table 3.

### (Example 5)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 7, in the same manner as in Example 1. The results are shown in Table 3.

### (Example 6)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 8, in the same manner as in Example 1. The results are shown in Table 3.

### (Example 7)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 9, in the same manner as in Example 1. The results are shown in Table 3.

### (Example 8)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 10, in the same manner as in Example 1. The results are shown in Table 3.

### (Example 9)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 13, in the same manner as in Example 1. The results are shown in Table 3.

### (Example 10)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 16, in the same manner as in Example 1. The results are shown in Table 3.

### (Comparative Example 1)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 1, in the same manner as in Example 1. The results are shown in Table 3.

### (Comparative Example 2)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 6, in the same manner as in Example 1. The results are shown in Table 3.

### (Comparative Example 3)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 11, in the same manner as in Example 1. The results are shown in Table 3.

### (Comparative Example 4)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 12, in the same manner as in Example 1. The results are shown in Table 3.

### (Comparative Example 5)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 14, in the same manner as in Example 1. The results are shown in Table 3.

### (Comparative Example 6)

The IL-6 adsorption rate, the rate of decrease in IL-6 adsorption performance, the IL-8 adsorption rate, the rate of decrease in IL-8 adsorption performance and the pH of the filling liquid after the high-pressure steam sterilization were determined for the biological component treatment material 15, in the same manner as in Example 1. The results are shown in Table 3.

### [Table 1]

**[Table 1]**

| Name of Biological Component Treatment Material | Name of Substrate | Chemical Structure of First Ligand | Chemical Structure of Second Ligand | Content of Amino Groups in First Ligand (mmol/g) | Content of Second Ligand (mmol/g) |
|---|---|---|---|---|---|
| Biological Component Treatment Material 1 | Substrate A | 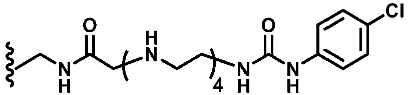 | None | 1.8 | 0 |
| Biological Component Treatment Material 2 | Substrate A | 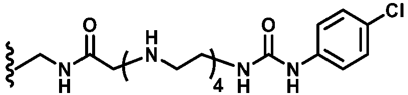 | 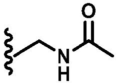 | 1.4 | 0.6 |
| Biological Component Treatment Material 3 | Substrate A | 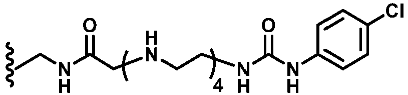 | 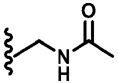 | 2.2 | 0.6 |
| Biological Component Treatment Material 4 | Substrate A | 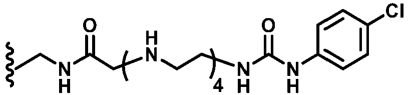 | 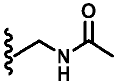 | 3.8 | 0.6 |
| Biological Component Treatment Material 5 | Substrate A | 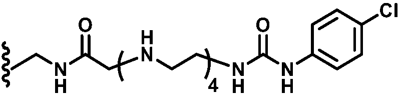 | 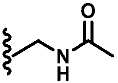 | 1.2 | 2.2 |
| Biological Component Treatment Material 6 | Substrate A | None | 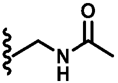 | 0 | 4.2 |
| Biological Component Treatment Material 7 | Substrate A | 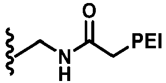 PEI : Polyethyleneimine | 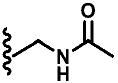 | 2.8 | 3 |
| Biological Component Treatment Material 8 | Substrate A | 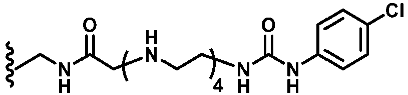 | 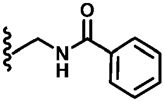 | 1.4 | 0.4 |
| Biological Component Treatment Material 9 | Substrate A | 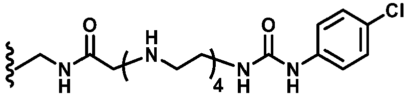 | 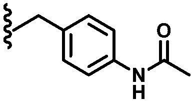 | 1.3 | 0.8 |
| Biological Component Treatment Material 10 | Substrate B | 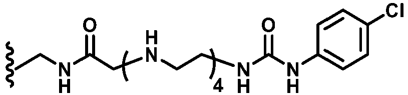 | 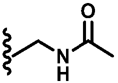 | 1.4 | 0.5 |

### [Table 2]

**[Table 2]**

| Name of Biological Component Treatment Material | Name of Substrate | Chemical Structure of First Ligand | Chemical Structure of Second Ligand | Content of Amino Groups in First Ligand (mmol/g) | Content of Second Ligand (mmol/g) |
|---|---|---|---|---|---|
| Biological Component Treatment Material 11 | Substrate A | 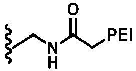 PEI : Polyethyleneimine | None | 2.2 | 0 |
| Biological Component Treatment Material 12 | Substrate A | 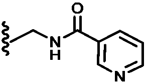 | 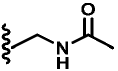 | 1.2 | 2.2 |
| Biological Component Treatment Material 13 | Substrate A | 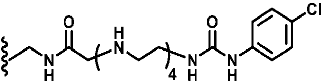 | 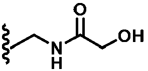 | 1.6 | 0.5 |
| Biological Component Treatment Material 14 | Substrate A | 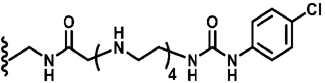 | 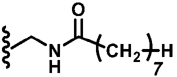 | 1.6 | 0.5 |
| Biological Component Treatment Material 15 | Substrate A | 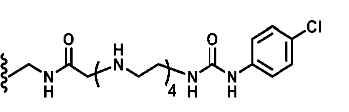 | 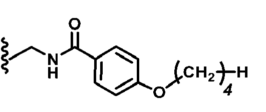 | 1.7 | 0.6 |
| Biological Component Treatment Material 15 | Substrate C | 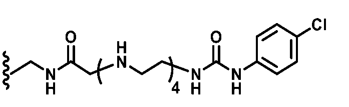 | 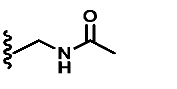 | 1.3 | 0.8 |

In Tables 1 and 2, the "Content of Amino Groups in First Ligand" represents the content of amino groups in the first ligand per 1 g of dry weight of the water-insoluble material, and the "Content of Second Ligand" represents the content of the second ligand per 1 g of dry weight of the water-insoluble material.

### [Table 3]

**[Table 3]**

| | Name of Biological Component Treatment Material | IL-6 adsorption rate (%) | IL-6 adsorption rate after high-pressure steam sterilization (%) | Rate of decrease in IL-6 adsorption performance (%) | IL-8 adsorption rate (%) | IL-8 adsorption rate after high-pressure steam sterilization (%) | Rate of decrease in IL-8 adsorption rate (%) | pH of filling liquid after high-pressure steam sterilization |
|---|---|---|---|---|---|---|---|---|
| Example 1 | Biological Component Treatment Material 2 | 69 | 82 | -19 | 89 | 95 | -7 | 4.2 |
| Example 2 | Biological Component Treatment Material 3 | 77 | 78 | -1 | 92 | 96 | -4 | 8.9 |
| Example 3 | Biological Component Treatment Material 4 | 54 | 50 | 7 | 61 | 55 | 10 | 8.6 |
| Example 4 | Biological Component Treatment Material 5 | 55 | 51 | 7 | 83 | 69 | 17 | 5.3 |
| Example 5 | Biological Component Treatment Material 7 | 58 | 55 | 5 | 72 | 66 | 8 | 7.3 |
| Example 6 | Biological Component Treatment Material 8 | 75 | 68 | 9 | 78 | 79 | -1 | 4.1 |
| Example 7 | Biological Component Treatment Material 9 | 59 | 59 | 0 | 83 | 81 | 2 | 4.3 |
| Example 8 | Biological Component Treatment Material 10 | 66 | 61 | 8 | 62 | 53 | 15 | 4.5 |
| Example 9 | Biological Component Treatment Material 13 | 85 | 79 | 7 | 89 | 81 | 9 | 4.4 |
| Example 10 | Biological Component Treatment Material 16 | 70 | 65 | 7 | 98 | 97 | 1 | 4.3 |
| Comparative Example 1 | Biological Component Treatment Material 1 | 56 | 41 | 27 | 88 | 58 | 34 | 3.8 |
| Comparative Example 2 | Biological Component Treatment Material 6 | 0 | 0 | 0 | 0 | 0 | 0 | 7.6 |
| Comparative Example 3 | Biological Component Treatment Material 11 | 52 | 39 | 25 | 81 | 55 | 32 | 4.1 |
| Comparative Example 4 | Biological Component Treatment Material 12 | 0 | 0 | 0 | 0 | 0 | 0 | 5.1 |
| Comparative Example 5 | Biological Component Treatment Material 14 | 3 | 3 | 0 | 44 | 34 | 23 | 4.8 |
| Comparative Example 6 | Biological Component Treatment Material 15 | 13 | 14 | -8 | 52 | 40 | 23 | 4.9 |

The results of Comparative Examples 1 and 3 in Table 3 have shown that conventional biological component treatment materials show a high rate of decrease in IL-6 adsorption performance and rate of decrease in IL-8 adsorption performance due to high-pressure steam sterilization, and thus are not suitable for high-pressure steam sterilization. In contrast, the results of Examples 1 to 5 have shown that a decrease in IL-6 adsorption performance and a decrease in IL-8 adsorption performance due to high-pressure steam sterilization can be reduced, and further that the acidification of the pH of the filling liquid after the high-pressure steam sterilization can be reduced, by containing the second ligand in addition to the first ligand. Further, the results of Example 5 and Comparative Example 4 have shown that the effect of the first ligand is exhibited within the range of the chemical structure of the general formula (I), regardless of the chemical structure of the first ligand. In addition, the results of Examples 6 and 7 as well as Comparative Examples 5 and 6 have shown that the effect of the second ligand is exhibited within the range of the chemical structure of the general formula (IIa) or (IIb), regardless of the chemical structure of the second ligand. Moreover, the results of Example 8 have shown that the effect of the substrate is exhibited regardless of the material of the substrate.

### Industrial Applicability

The biological component treatment material according to the present invention can be suitably used as an adsorption material for use in a biological component treatment column intended to treat an unwanted biological component, particularly for use in a blood purification column for the treatment of an inflammatory disease.

## Claims

1. A biological component treatment material, comprising a water-insoluble material comprising:
a first ligand having an amino group and an amide group;
a second ligand having a hydrocarbon group optionally substituted with a hydroxy group, and an amide group; and
a substrate;
wherein said first ligand has a structure of the following general formula (I) and is bound to said substrate as indicated by the following general formula (I), and
wherein said second ligand has a structure of the following general formula (IIa) or (IIb) and is bound to said substrate as indicated by the following general formula (IIa) or (IIb): 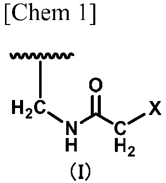 [wherein the wavy line represents a point of attachment to said substrate; and X represents a chemical structure containing an amino group as a partial structure, and optionally containing a phenyl group] 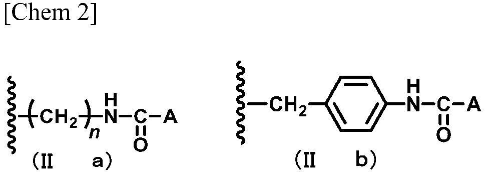 [wherein the wavy line represents a point of attachment to said substrate; A represents an alkyl group having from 1 to 6 carbon atoms or a phenyl group, optionally substituted with a hydroxy group; and n represents an integer from 1 to 6].

2. The biological component treatment material according to claim 1, comprising said second ligand at a content of from 0.3 to 5.0 mmol per 1 g of dry weight of said water-insoluble material.

3. The biological component treatment material according to claim 1 or 2, comprising said amino group in said first ligand at a content of from 1.0 to 4.0 mmol per 1 g of dry weight of said water-insoluble material.

4. A biological component treatment column, comprising the biological component treatment material according to any one of claims 1 to 3.
